# EUROPEAN PATENT APPLICATION

(11) **EP 4 303 223 A1**
(43) Date of publication of application: **10.01.2024**
(21) Application number: 23183619.8
(22) Date of filing: 05.07.2023
(51) Int. Cl.: C07F 17/00, C08F 4/00, C08F 210/00

(54) **NOVEL TRANSITION METAL COMPOUND, TRANSITION METAL CATALYST COMPOSITION CONTAINING THE SAME, AND METHOD FOR PRODUCING COPOLYMER OF ETHYLENE AND ALPHA-OLEFIN USING THE SAME**

(30) Priority: 05.07.2022 KR 20220082230
(71) Applicant: Hanwha TotalEnergies Petrochemical Co., Ltd., Seosan-si, Chungcheongnam-do 31900 (KR)
(72) Inventor: Kim, Young Seub, 31909 Chungcheongnam-do (KR); Kim, Ho Yeun, 03709 Seoul (KR); Lee, Do Hoon, 06603 Seoul (KR); Lee, Young Joo, 16295 Gyeonggi-do (KR); Lee, Dong Geun, 31909 Chungcheongnam-do (KR)
(74) Representative: Simmons & Simmons LLP (Munich)

(57) **Abstract**

The present invention relates to a novel transition metal compound, a transition metal catalyst composition having high catalytic activity for producing a copolymer of ethylene and α-olefin containing the same, a method for producing a copolymer of ethylene and α-olefin using the same, and a copolymer of ethylene and α-olefin produced using the same.

## Description

### TECHNICAL FIELD

The following disclosure relates to a novel transition metal compound, a transition metal catalyst composition for producing a copolymer of ethylene and α-olefin containing the same, a method for producing a copolymer of ethylene and α-olefin using the same, and a copolymer of ethylene and α-olefin produced using the transition metal compound as a catalyst.

### BACKGROUND

In the related art, a so-called Ziegler-Natta catalyst system composed of a titanium or vanadium compound as a main catalyst component and an alkylaluminum compound as a cocatalyst component has been generally used to produce an ethylene homopolymer and a copolymer of ethylene and α-olefin. The Ziegler-Natta catalyst system exhibits high activity for ethylene polymerization, but has disadvantages in that a molecular weight distribution of the generally produced polymer is broad due to heterogeneous catalytic active sites, and in particular, a uniform composition distribution is not implemented in the copolymer of ethylene and α-olefin.

Subsequently, various studies have been conducted on, as a homogeneous catalyst having a single catalyst active site, a metallocene catalyst system that may produce polyethylene having a narrow molecular weight distribution and a uniform composition distribution compared to the existing Ziegler-Natta catalyst system and is composed of a metallocene compound of a transition metal of Group 4 in the periodic table, such as zirconium, hafnium, etc., and methylaluminoxane as a cocatalyst. The metallocene compound, a cyclopentadienyl-based catalyst having a different substitution pattern, is currently actively used industrially, and is also used for the production of polyethylene as well as polypropylene.

However, it is difficult to obtain a high molecular weight polymer with the above catalyst system. That is, it is known that when a solution polymerization method performed at a high temperature is applied, the polymerization activity is rapidly reduced and β-dehydrogenation reaction is dominant, which is unsuitable for producing a high molecular weight polymer.

A transition metal catalyst in which transition metals are linked in a ring form has been announced as a catalyst capable of producing a polymer having high catalytic activity and high molecular weight in homopolymerization of ethylene or copolymerization of ethylene and α-olefin under solution polymerization conditions.

US 6,313,240 discloses a catalyst system containing: a cyclopentadienyl ligand or an aromatic-fused and substituted cyclopentadienyl ligand; an aromatic-fused and substituted cyclopentadienyl ligand; and a hafnium organometallic compound having a bridge connecting two cyclopentadienyl ligands.

In addition, US 6,559,253 discloses a structure in which a diphenyl-substituted bridge and one cyclopentadiene ligand are linked to fluorene with no substituent as an example. US 6,300,433 discloses a structure of one or more substituted cyclopentadienyl-fluorenyl ligands having a substituted diphenyl methylene bridge.

In the case of these catalysts, the reactivity with α-olefins is significantly improved due to a reduced steric hindrance effect of the catalyst itself, but there are many difficulties in commercial use. Therefore, it is important to secure a more competitive catalyst system in terms of the required characteristics of commercial catalysts based on economic feasibility, that is, excellent high-temperature activity, excellent reactivity with α-olefins, and the ability to produce polymers having a high molecular weight.

### SUMMARY

An embodiment of the present invention is directed to providing a novel transition metal compound.

Another embodiment of the present invention is directed to providing a transition metal catalyst composition containing the transition metal compound capable of producing a high molecular weight copolymer of ethylene and α-olefin.

Still another embodiment of the present invention is directed to providing an industrially economical and easy-to-use method for producing a copolymer of ethylene and α-olefin using a catalyst composition containing the transition metal compound.

In order to achieve the above object, as a result of conducting studies, the present inventors have found that when an active site of a compound used as a catalyst is stabilized, a high molecular weight copolymer of ethylene and α-olefin may be produced in a high-temperature solution polymerization, thereby completing the present invention.

In one general aspect, there is provided a transition metal compound represented by the following Chemical Formula 1:

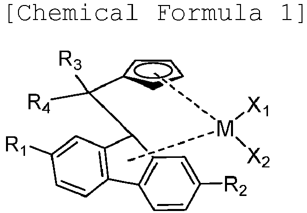

wherein
M is a Group 4 transition metal;
R₁ and R₂ are each independently (C6-C20) aryl (C1-C20) alkyl unsubstituted or substituted with (C1-C10)alkyl;
R₃ and R₄ are each independently (C6-C20) aryl unsubstituted or substituted with (C1-C10)alkyl;
X₁ and X₂ are each independently halogen, (C1-C20) alkyl, (C3-C20)cycloalkyl, (C6-C20)aryl, (C6-C20) aryl (C1-C20) alkyl, ((C1-C20)alkyl(C6-C20)aryl)(C1-C20)alkyl, (C1-C20)alkoxy, (C6-C20)aryloxy, (C1-C20)alkyl(C6-C20)aryloxy, (C1-C20)alkoxy(C6-C20) aryloxy, -OSiRₐR_{b}R_{c}, -SR_{d}, -NRₑR_{f}, -PR_{g}Rₕ, or (C1-C20)alkylidene;
Rₐ to R_{d} are each independently (C1-C20) alkyl, (C6-C20) aryl, (C6-C20)aryl(C1-C20)alkyl, (C1-C20)alkyl(C6-C20)aryl, or (C3-C20)cycloalkyl;
Rₑ to Rₕ are each independently (C1-C20) alkyl, (C6-C20) aryl, (C6-C20)aryl(C1-C20)alkyl, (C1-C20)alkyl(C6-C20)aryl, (C3-C20)cycloalkyl, tri(C1-C20)alkylsilyl, or tri(C6-C20)arylsilyl; and
when one of X₁ and X₂ is (C1-C20)alkylidene, the other one is absent.

Specifically, in Chemical Formula 1, M may be Ti, Zr, or Hf; R₁ and R₂ may be each independently (C6-C20) aryl (C1-C20) alkyl; R₃ and R₄ may be each independently (C6-C12)aryl unsubstituted or substituted with (C1-C5) alkyl; and X₁ and X₂ may be each independently halogen, (C1-C20)alkyl, (C6-C20)aryl, or (C6-C20)aryl(C1-C20)alkyl.

In addition, in Chemical Formula 1, M may be Hf; R₁ and R₂ may be each independently (C6-C12)aryl(C1-C10)alkyl; R₃ and R₄ may be each independently (C6-C12)aryl; and X₁ and X₂ may be each independently halogen, (C1-C10)alkyl, (C6-C12)aryl, or (C6-C12)aryl(C1-C10)alkyl.

More specifically, according to an exemplary embodiment of the present invention, in Chemical Formula 1, M may be Hf; R₃ and R₄ may be each independently phenyl, X₁ and X₂ may be each independently methyl, benzyl, or Cl; and R₁ and R₂ may be each independently represented by the following Chemical Formula 2:

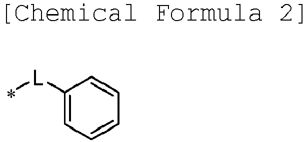

wherein
L is linear or branched (C1-C10)alkylene.

The transition metal compound according to an exemplary embodiment of the present invention may be [1-(η5-cyclopentadien-1-yl)-1-(η5-2,7-di-(2-phenylpropan-2-yl)fluorenyl)-1,1-diphenyl methane]hafnium dichloro, [1-(η5-cyclopentadien-1-yl)-1-(η5-2,7-di-(2-phenylpropan-2-yl)fluorenyl)-1,1-diphenyl methane]hafnium dibenzyl, or [1-(η5-cyclopentadien-1-yl)-1-(η5-2,7-di-(2-phenylpropan-2-yl)fluorenyl)-1,1-diphenyl methane]hafnium dimethyl.

In another general aspect, there is provided a transition metal catalyst composition for producing a copolymer of ethylene and α-olefin containing: the transition metal compound according to an exemplary embodiment of the present invention; and a cocatalyst selected from an aluminum compound, a boron compound, and a mixture thereof.

The aluminum compound used as the cocatalyst may be one or two or more selected from aluminoxane and organic aluminum.

In still another general aspect, a method for producing a copolymer of ethylene and α-olefin includes: a) mixing the transition metal catalyst composition according to an exemplary embodiment of the present invention, ethylene, and an α-olefin comonomer; and b) performing a copolymerization reaction at a temperature of 110 to 170°C.

The α-olefin copolymerized with ethylene may be one or two or more selected from propylene, 1-butene, 1-pentene, 4-methyl-1-pentene, 1-hexene, 1-heptene, 1-octene, 1-decene, 1-undecene, 1-dodecene, 1-tetradecene, 1-hexadecene, 1-octadecene, 1-eicosene, cyclopentene, cyclohexene, norbornene, phenylnorbornene, styrene, α-methylstyrene, p-methylstyrene, and 3-chloromethylstyrene.

Specifically, the step b) may be performed at a temperature of 120 to 160°C and a pressure of 10 to 100 bar.

In addition, the method for producing a copolymer of ethylene and α-olefin may be performed in a C5-C12 aliphatic hydrocarbon solvent.

In still another general aspect, there is provided a copolymer of ethylene and α-olefin produced using the transition metal compound according to an exemplary embodiment of the present invention as a catalyst.

Other features and aspects will be apparent from the following detailed description, the drawings, and the claims.

### DETAILED DESCRIPTION OF EMBODIMENTS

Hereinafter, a novel transition metal compound of the present invention, a transition metal catalyst composition containing the same, and a method for producing a copolymer of ethylene and α-olefin using the same will be described in detail.

Unless the context clearly indicates otherwise, singular forms used in the present invention may be intended to include plural forms.

The expression "comprise(s)" described in the present invention is intended to be an open-ended transitional phrase having an equivalent meaning to "include(s)", "contain(s)", "have (has)", and "are (is) characterized by", and does not exclude elements, materials, or steps, all of which are not further recited herein.

The term "alkyl" described in the present invention refers to a monovalent linear or branched saturated hydrocarbon radical composed of only carbon and hydrogen atoms. Examples of the alkyl radical include, but are not limited to, methyl, ethyl, propyl, isopropyl, butyl, isobutyl, t-butyl, pentyl, hexyl, octyl, and nonyl.

The term "aryl" described in the present invention refers to an organic radical derived from an aromatic hydrocarbon by removal of one hydrogen, includes a monocyclic or fused ring system having suitably 4 to 7 ring atoms, and preferably 5 or 6 ring atoms in each ring, and even includes a form in which a plurality of aryls are linked by a single bond. The fused ring system may include an aliphatic ring such as a saturated or partially saturated ring, and necessarily includes at least one aromatic ring. In addition, the aliphatic ring may include nitrogen, oxygen, sulfur, carbonyl, and the like, in a ring. Specific examples of the aryl radical include, but are not limited to, phenyl, naphthyl, biphenyl, indenyl, fluorenyl, phenanthrenyl, anthracenyl, triphenylenyl, pyrenyl, chrysenyl, naphthacenyl, and 9,10-dihydroanthracenyl.

The term "cycloalkyl" described in the present invention refers to a monovalent saturated carbocyclic radical composed of one or more rings. Examples of the cycloalkyl radical include, but are not limited to, cyclopropyl, cyclobutyl, cyclopentyl, cyclohexyl, and cycloheptyl.

The term "halo" or "halogen" described in the present invention refers to a fluorine, chlorine, bromine, or iodine atom.

The term "alkoxy" described in the present invention refers to -O-(alkyl) including -OCH₃, -OCH₂CH₃, -O(CH₂)₂CH₃, -O(CH₂)₃CH₃, -O(CH₂)₄CH₃, -O(CH₂)₅CH₃, and the like, where the "alkyl" is as defined above.

The term "aryloxy" described in the present invention refers to an -O-aryl radical, where the "aryl" is as defined above.

The term "alkylidene" described in the present invention refers to a linear or branched saturated divalent hydrocarbon group having a valence of 2 on a single common carbon atom.

The present invention provides a transition metal compound represented by the following Chemical Formula 1:

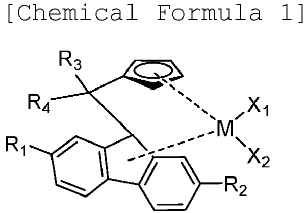

wherein
M is a Group 4 transition metal;
R₁ and R₂ are each independently (C6-C20) aryl (C1-C20) alkyl unsubstituted or substituted with (C1-C10)alkyl;
R₃ and R₄ are each independently (C6-C20)aryl unsubstituted or substituted with (C1-C10)alkyl;
X₁ and X₂ are each independently halogen, (C1-C20)alkyl, (C3-C20)cycloalkyl, (C6-C20)aryl, (C6-C20)aryl(C1-C20)alkyl, ((C1-C20)alkyl(C6-C20)aryl)(C1-C20)alkyl, (C1-C20)alkoxy, (C6-C20)aryloxy, (C1-C20)alkyl(C6-C20)aryloxy, (C1-C20)alkoxy(C6-C20) aryloxy, -OSiRₐR_{b}R_{c}, -SR_{d}, -NRₑR_{f}, -PR_{g}Rₕ, or (C1-C20)alkylidene;
Rₐ to R_{d} are each independently (C1-C20)alkyl, (C6-C20)aryl, (C6-C20)aryl(C1-C20)alkyl, (C1-C20)alkyl(C6-C20)aryl, or (C3-C20)cycloalkyl;
Rₑ to Rₕ are each independently (C1-C20) alkyl, (C6-C20) aryl, (C6-C20)aryl(C1-C20)alkyl, (Cl-C20)alkyl(C6-C20)aryl, (C3-C20)cycloalkyl, tri(C1-C20)alkylsilyl, or tri(C6-C20)arylsilyl; and
when one of X₁ and X₂ is (C1-C20)alkylidene, the other one is absent.

The transition metal compound represented by Chemical Formula 1 is a compound having a structure in which transition metals of Group 4 in the periodic table as central metals are linked by a cyclopentadienyl group in which electrons are rich and widely delocalized and a fluorenyl group substituted with an arylalkyl substituent at positions 2 and 7, and the cyclopentadienyl group and the fluorenyl group are linked by carbon, and may exhibit excellent catalytic activity in homopolymerization of ethylene or copolymerization of ethylene and α-olefin because the catalytic active site may be stabilized due to the fluorenyl group substituted with an arylalkyl substituent at positions 2 and 7. Furthermore, when the transition metal compound is used in a solution polymerization process performed at a high temperature, a high molecular weight ethylene homopolymer or copolymer of ethylene and α-olefin may be produced.

Specifically, in Chemical Formula 1, M may be Ti, Zr, or Hf; R₁ and R₂ may be each independently (C6-C20) aryl (C1-C20) alkyl; R₃ and R₄ may be each independently (C6-C12)aryl unsubstituted or substituted with (C1-C5)alkyl; and X₁ and X₂ may be each independently halogen, (C1-C20)alkyl, (C6-C20)aryl, or (C6-C20)aryl(C1-C20)alkyl.

According to an exemplary embodiment, in Chemical Formula 1, M may be Hf; R₁ and R₂ may be each independently (C6-C12)aryl(C1-C10)alkyl; R₃ and R₄ may be each independently (C6-C12)aryl; and X₁ and X₂ may be each independently halogen, (C1-C10)alkyl, (C6-C12)aryl, or (C6-C12)aryl(C1-C10)alkyl.

In addition, according to an exemplary embodiment, in Chemical Formula 1, M may be Hf; R₁ and R₂ may be each independently (C6-C12)aryl(C1-C5)alkyl; R₃ and R₄ may be each independently (C6-C12)aryl; and X₁ and X₂ may be each independently halogen, (C1-C5)alkyl, (C6-C12)aryl, or (C6-C12)aryl(C1-C5)alkyl.

More specifically, according to an exemplary embodiment of the present invention, in Chemical Formula 1, M may be Hf; R₃ and R₄ may be each independently phenyl, X₁ and X₂ may be each independently methyl, benzyl, or Cl; and R₁ and R₂ may be each independently represented by the following Chemical Formula 2:

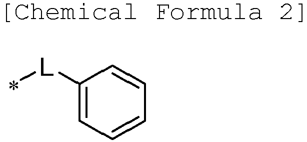

wherein
L is linear or branched (C1-C10)alkylene.

More specifically, in Chemical Formula 2, L may be linear or branched (C1-C5)alkylene.

The transition metal compound according to an exemplary embodiment of the present invention may be [1-(η5-cyclopentadien-1-yl)-1-(η5-2,7-di-(2-phenylpropan-2-yl)fluorenyl)-1,1-diphenyl methane]hafnium dichloro, [1-(η5-cyclopentadien-1-yl)-1-(η5-2,7-di-(2-phenylpropan-2-yl)fluorenyl)-1,1-diphenyl methane]hafnium dibenzyl, or [1-(η5-cyclopentadien-1-yl)-1-(η5-2,7-di-(2-phenylpropan-2-yl)fluorenyl)-1,1-diphenyl methane]hafnium dimethyl.

Meanwhile, in order for the transition metal compound according to an exemplary embodiment of the present invention to be an active catalyst component used in the production of the copolymer of ethylene and α-olefin, preferably, the X₁ and X₂ ligands of the transition metal compound of Chemical Formula 1 are extracted, such that the central metal may be cationized and an aluminum compound, a boron compound, or a mixture thereof that may act as a counter ion having a weak binding force, that is, an anion, may be used as a cocatalyst.

Accordingly, the present invention provides a transition metal catalyst composition for producing a copolymer of ethylene and α-olefin containing: the transition metal compound according to an exemplary embodiment of the present invention; and a cocatalyst selected from an aluminum compound, a boron compound, and a mixture thereof.

In the transition metal catalyst composition for producing a copolymer of ethylene and α-olefin according to an exemplary embodiment of the present invention, the aluminum compound used as the cocatalyst may be one or two or more selected from aluminoxane, organoaluminum, and organoaluminum oxide compounds. Specifically, the aluminum compound may be one or two or more selected from an aluminoxane compound of the following Chemical Formula 3 or 4, an organoaluminum compound of the following Chemical Formula 5, and an organoaluminum oxide compound of the following Chemical Formula 6 or 7:

[Chemical Formula 3] (-Al(R¹¹)-O-)ₘ

[Chemical Formula 4] (R¹¹)₂Al-(-O(R¹¹)-)_{q}-(R¹¹)₂

[Chemical Formula 5] (R¹²)ᵣAl (E) ₃₋ᵣ

[Chemical Formula 6] (R¹³)₂AlOR¹⁴

[Chemical Formula 7] R¹³Al (OR¹⁴) ₂

wherein
R¹¹ is (C1-C20) alkyl;
R¹² and R¹³ are each independently (C1-C20) alkyl;
E is hydrogen or halogen;
R¹⁴ is (C1-C20) alkyl or (C6-C20) aryl;
m and q are each independently an integer of 5 to 20; and
r is an integer of 1 to 3.

Specifically, in Chemical Formulas 3 and 4, R¹¹ may be methyl or isobutyl.

Specific examples of a compound that may be used as the aluminum compound include: aluminoxane compounds such as methylaluminoxane, modified methylaluminoxane, and tetraisobutylaluminoxane; and organoaluminum compounds, for example, trialkylaluminums such as trimethylaluminum, triethylaluminum, tripropylaluminum, triisobutylaluminum, trihexylaluminum, and trioctylaluminum, dialkylaluminum chlorides such as dimethylaluminum chloride, diethylaluminum chloride, dipropylaluminum chloride, diisobutylaluminum chloride, and dihexylaluminum chloride, alkylaluminum dichlorides such as methylaluminum dichloride, ethylaluminum dichloride, propylaluminum dichloride, isobutylaluminum dichloride, and hexylaluminum dichloride, and dialkylaluminum hydrides such as dimethylaluminum hydride, diethylaluminum hydride, dipropylaluminum hydride, diisobutylaluminum hydride, and dihexylaluminum hydride.

More preferably, the aluminum compound may be one or two or more selected from methylaluminoxane, modified methylaluminoxane, tetraisobutylaluminoxane, trimethylaluminum, triethylaluminum, trioctylaluminum, and triisobutylaluminum.

The boron compound that may be used as the cocatalyst in the present invention may be selected from boron compounds represented by the following Chemical Formulas 8 to 10:

[Chemical Formula 8] B (R²¹)₃

[Chemical Formula 9] [R²²]⁺[B(R²¹)₄]⁻

[Chemical Formula 10] [(R²³)ₚZH]⁺[B(R²¹)₄]⁻

wherein
B is a boron atom;
Z is a nitrogen or phosphorus atom;
R²¹ is phenyl;
the phenyl may be further substituted with 3 to 5 substituents selected from fluoro, (C1-C20)alkyl unsubstituted or substituted with fluoro, and (C1-C20)alkoxy unsubstituted or substituted with fluoro;
R²² is a (C5-C7)aryl radical, a (C1-C20)alkyl(C6-C20)aryl radical, or a (C6-C20)aryl(Cl-C20)alkyl radical;
R²³ is a (C1-C50)alkyl radical or an anilinium radical substituted with two (C1-C10)alkyls together with a nitrogen atom; and
p is an integer of 2 or 3.

More specifically, R²² may be a triphenylmethylium radical.

Preferably, the boron compound used as the cocatalyst may be one or two or more selected from dimethylphenylammonium tetraphenylborate, trityl tetraphenylborate, dimethylphenylammonium tetrakis(pentafluorophenyl)borate, trityl tetrakis(pentafluorophenyl)borate, trimethylammonium tetraphenylborate, triethylammonium tetraphenylborate, tripropylammonium tetraphenylborate, tributylammonium tetraphenylborate, trimethylammonium tetrakis(pentafluorophenyl)borate, triethylammonium tetrakis(pentafluorophenyl)borate, tripropylammonium tetrakis(pentafluorophenyl)borate, tributylammonium tetrakis(pentafluorophenyl)borate, anilinium tetraphenylborate, anilinium tetrakis(pentafluorophenyl)borate, pyridinium tetrakis(pentafluorophenyl)borate, and silver tetrakis(pentafluorophenyl)borate.

Meanwhile, the cocatalyst may serve as a scavenger that removes impurities that act as poisons to the catalyst in the reactant.

In an exemplary embodiment of the present invention, when the aluminum compound and the boron compound are used as cocatalysts, as a preferred range of a ratio between the transition metal compound of the present invention and the cocatalysts, a molar ratio of transition metal (M):aluminum atom (Al):boron atom (B) may be 1: (10 to 3,000):(1 to 100), and more preferably 1: (100 to 1,000): (3 to 10) .

When the ratio between the transition metal compound of the present invention and the cocatalysts is within the above range, the transition metal compound may be fully activated, and thus the catalytic activity of the transition metal compound may be excellent, but the above ratio is not limited thereto, and the range of the ratio may vary depending on the conditions and purpose of the reaction.

The present invention provides a method for producing a copolymer of ethylene and α-olefin, the method including: a) mixing the transition metal catalyst composition according to an exemplary embodiment of the present invention, ethylene, and an α-olefin comonomer; and b) performing a copolymerization reaction at a temperature of 110 to 170°C.

In addition, the present invention provides a method for producing an ethylene homopolymer as well as the method for producing a copolymer, and the method for producing an ethylene homopolymer may be performed in the same manner as in the method for producing a copolymer except that only ethylene is used instead of the comonomer.

Specifically, the step b) may be performed at a temperature of 120 to 165°C and a pressure of 10 to 100 bar, and preferably, may be performed at a temperature of 130 to 160°C and a pressure of 15 to 50 bar.

In the method for producing a copolymer of ethylene and α-olefin, the α-olefin may be one or two or more selected from (C3-C18)α-olefin, (C5-C20)cycloolefin, styrene, and a derivative of styrene, (C3-C18)α-olefin may be one or two or more selected from the group consisting of propylene, 1-butene, 1-pentene, 4-methyl-1-pentene, 1-hexene, 1-heptene, 1-octene, 1-decene, 1-undecene, 1-dodecene, 1-tetradecene, 1-hexadecene, and 1-octadecene, (C5-C20)cycloolefin may be one or two or more selected from the group consisting of cyclopentene, cyclohexene, norbornene, and phenylnorbornene, and styrene and a derivative thereof may be one or two or more selected from styrene, α-methylstyrene, p-methylstyrene, and 3-chloromethylstyrene. More specifically, the α-olefin may be one or two or more selected from 1-butene, 1-hexene, 1-octene, and 1-decene, but is not limited thereto.

The method for producing an ethylene homopolymer or a copolymer of ethylene and α-olefin using the transition metal catalyst composition according to an exemplary embodiment of the present invention may be performed by bringing the transition metal catalyst, cocatalyst, α-olefin comonomer, and ethylene in contact with each other in the presence of an appropriate organic solvent. In this case, the transition metal catalyst, the cocatalyst, and the α-olefin comonomer may be separately put in a reactor or may be mixed in advance and then put in the reactor.

In addition, the method for producing a copolymer of ethylene and α-olefin may be performed in a C5-C12 aliphatic hydrocarbon solvent, and specifically, the C5-C12 aliphatic hydrocarbon solvent may be one or two or more selected from butane, isobutane, pentane, hexane, heptane, octane, isooctane, nonane, decane, dodecane, cyclohexane, and methylcyclohexane, and preferably, may be hexane, cyclohexane, or a mixture thereof.

The method for producing a copolymer of ethylene and α-olefin according to an exemplary embodiment of the present invention is a more economical method because toluene, which is a co-solvent commonly used in production of a copolymer, is not used, resulting in simplicity of a toluene solvent removal step in the production process.

The present invention provides an ethylene homopolymer or a copolymer of ethylene and α-olefin produced using the transition metal catalyst composition according to an exemplary embodiment of the present invention, and the produced homopolymer or copolymer may be easily and economically used for producing from an elastomer to high-density polyethylene (HDPE) having a density of 0.850 g/mL to 0.910 g/mL and a melt flow rate of 0.001 to 20 g/10 min.

In addition, hydrogen may be used as a molecular weight modifier to control the molecular weight when producing the ethylene homopolymer or the copolymer of ethylene and α-olefin according to the present invention, and a weight average molecular weight of the homopolymer or copolymer produced may be 5,000 to 1,000,000 g/mol, specifically, 10,000 to 800,000 g/mol, and more specifically, 30,000 to 500,000 g/mol.

The catalyst composition according to an exemplary embodiment of the present invention is significantly suitable for a high-temperature solution polymerization process because it may maintain a uniform shape in a polymerization reactor, and may also be applied in a slurry polymerization process or a gas phase polymerization process in the form of a heterogeneous catalyst obtained by supporting the catalyst and the composition containing the catalyst on a porous metal oxide support.

The present invention provides a high molecular weight ethylene homopolymer or copolymer of ethylene and α-olefin produced using the transition metal compound according to an exemplary embodiment as a catalyst.

Hereinafter, the novel transition metal compound according to the present invention, the transition metal catalyst composition containing the same, and the method for producing a copolymer of ethylene and α-olefin using the same will be described in more detail with reference to specific Examples.

All of the following synthesis reactions were performed in an inert atmosphere such as nitrogen or argon using the standard Schlenk technique and the glove box technique.

In addition, solvents for synthesis such as tetrahydrofuran (THF), n-hexane, n-pentane, diethyl ether, and methylene chloride (CH₂C₁₂) were passed through an activated alumina column to remove moisture and then used as being preserved on an activated molecular sieve. Unless specifically stated otherwise, most reagents used were purchased from Sigma-Aldrich, Tokyo Chemical Industry Co., Ltd. (TCI), Alfa Aesar, and Strem Chemicals, Inc.

¹H NMR analysis of the synthesized compound was performed using a Bruker 500 MHz at room temperature.

The molecular weight and copolymerizability of ethylene homopolymers or copolymers of ethylene and α-olefin were analyzed according to the following methods.

Melt flow rate (MFR) analysis was performed by melting plastic at 190°C and measuring a weight of an extrudate flowing through a capillary (orifice) having a certain size at loads of 2.16, 5, and 21.6 kg for 10 minutes. MFR was expressed as a melt index (MI) (g/10 min) according to the ASTM D1238 measurement standard. When MI was low, the molecular weight increased and flowability was reduced, such that processability was reduced and physical properties were improved.

GPC analysis relates to molecular weight averages (Mw and Mn), a molecular weight distribution (MWD), and its broadness of the polymer, and is performed by connecting 3 PLgel Olexis columns (300 × 7.5 mm, Polymer Laboratories) in series at 160°C on PLXT-20 High-Speed GPC Polymer Analysis System (including pump, refractive index detector, and viscosity detector, Polymer Laboratories) by high temperature size-exclusion chromatography (HTSEC). 1,2,4-Trichlorobenzene containing butylated hydroxytoluene (0.5 g/L) and Irganox 1010 (20 mg/L) was used as an eluent at a flow rate of 1.0 mL/min. The molecular weight was calculated based on polyethylene standard (Mp = 5,310 to 1,510,000 g/mol, Polymer Laboratories). A PL-XT-220 robotic sample handling system (Polymer Laboratories) was used as an automatic sampler. The analyzed concentration of the sample was 2 to 4 polymer mg/TCB mL.

In the density range of 0.850 to 0.910 g/mL, the closer the density was to 0.85, the higher the copolymerizability was. The weight of the resin per unit volume was measured to measure the density. The density gradient method in which calibration curves for standard column density and column height were created was used, and the analysis was performed according to the ASTM D1505 (KS M 3016) measurement standard.

A DSC experiment in which the temperature was controlled was performed with Q2000 DSC (TA Instruments) calibrated with indium, tin, and zinc and operated in modulation mode according to ISO 11357-1. 5 mg of a sample was placed in an aluminum pan, the temperature was raised to the initial temperature of 180°C, and then the temperature was lowered to - 88°C at 10°C/min as in standard DSC. Thereafter, the temperature was raised at a heating rate of 2°C/min under controlling the temperature at 0.32°C every 60 seconds. A glass transition temperature was measured with a reversible heat flow thermogram showing a point of inversion at transition. The higher the copolymerizability was, the lower the Tm was measured.

### [Preparation Example 1] Preparation of [1-(η5-Cyclopentadien-1-yl)-1-(η5-2,7-di-(2-phenylpropanyl)-fluorenyl)-1,1-diphenyl methane]hafnium dichloro (Compound 1)

### Step 1: Preparation of 2,7-Dibenzoyl-fluorene (Compound 1-a)

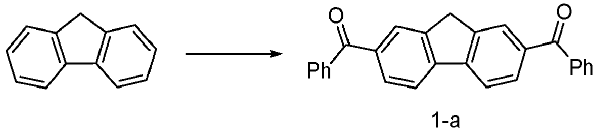

AlCl₃ (17.6 g, 132.3 mmol), DCM (120 mL), and fluorene (10 g, 60.2 mmol) were put in a Schlenk flask, benzoyl chloride (13.9 mL, 120.4 mmol) was added at 0°C, and then stirring was performed at room temperature for 18 hours. After the reaction was terminated by slowly adding ice and water, DCM was added to extract an organic layer. The organic layer was collected, dried over MgSO₄, and filtered under reduced pressure, and the resultant was recrystallized using Ether/Hex, thereby obtaining Compound 1-a (20 g, yield 91%) as a yellow solid.

¹H NMR (500 MHz, Chloroform-d) δ 7.90 (s, 2H), 7.88 (d, *J* = 7.7 Hz, 2H), 7.85 (d, *J* = 7.5 Hz, 2H) 7.83 (m, 4H), 7.60 (d, *J* = 7.7 Hz, 2H), 7.50 (m, 4H), 4.05 (s, 2H)

### Step 2: Preparation of 2,7-Di-(2-phenylpropan-2-yl)-fluorene (Compound 1-b)

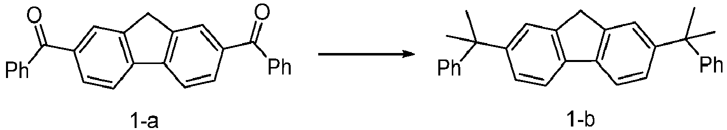

Compound 1-a (8.2 g, 22 mmol), toluene (109 mL), and acetic acid (0.3 mL, 5.47 mmol) were put in a Schlenk flask, trimethylaluminum (109 mL, 219 mmol, 2.0 M in Hex) was added, and reflux was performed at 100°C for 6 hours. The reaction was terminated by adding 1 N HCl and ice, and an organic layer was extracted with ether three times. The organic layers were collected, dried over MgSO₄, and filtered under reduced pressure, and the resultant was purified by a silica column (solvent Hex), thereby obtaining Compound 1-b (8.5 g, yield 96 %) as a yellow solid.

¹H NMR (500 MHz, Chloroform-d) δ 7.61 (d, *J* = 7.7 Hz, 2H), 7.38 (s, 2H), 7.23 (m, 8H), 7.21 (m, 2H), 7.17 (m, 2H), 3.78 (s, 2H), 1.72 (s, 12H).

### Step 3: Preparation of 1-(2,4-Cyclopentadien-1-yl)-1-(2,7-di-(2-phenylpropan-2-yl)-fluorenyl)-1,1-diphenyl methane (Compound 1-c)

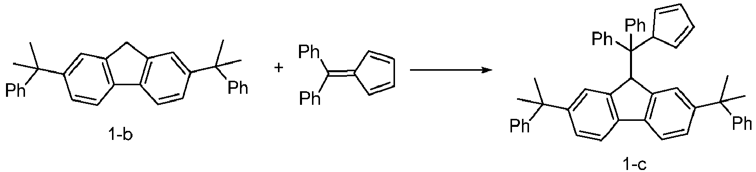

Compound 1-b (12 g, 43.1 mmol) was dissolved in 87 mL of THF, nBuLi (1.6 M in Hex, 27.1 mL, 43.1 mmol) was added, and stirring was performed at room temperature for 3 hours. 6,6-Diphenylfulvene (10 g, 43.1 mmol) was added, the reaction solution was stirred for 16 hours, and then the reaction was terminated by adding an aqueous NH₄Cl solution (40 mL). The product was extracted into an organic layer, the organic layer was dried over MgSO₄ and filtered under reduced pressure, and then the resulting yellow solid was washed with ethanol, thereby obtaining Compound 1-c (24 g, yield 94%) as a white solid.

¹H NMR (500 MHz, Chloroform-*d*) δ 7.02∼7.30 (m, 28H), 6.21 (s, 2H), 5.41 (s, 1H), 2.83 (br s, 1H), 1.53 (s, 12H).

### Step 4: Preparation of [1-(η5-Cyclopentadien-1-yl)-1-(η5-2,7-di-(2-phenylpropan-2-yl)-fluorenyl)-1,1-diphenyl methane]hafnium dichloro (Compound 1)

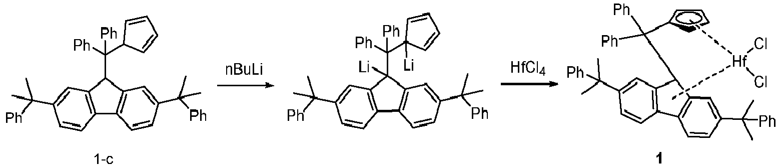

Compound 1-c (2.5 g, 3.95 mmol) was dissolved in 39 mL of ether, nBuLi (1.6 M in Hex, 5.43 mL, 8.69 mmol) was added, and stirring was performed for 16 hours. Ether was removed by vacuum drying, and then Hex was added and decanted under reduced pressure. Lithium (2.5 g, 3.89 mmol) and HfCl₄ (1.24 g, 3.89 mmol) that were weighed in a glove box were dissolved in 35 mL of ether, stirring was performed at room temperature for 16 hours, and then ether was removed by vacuum drying. 80 mL of toluene was added, heating was performed at 50°C for 2 hours, produced LiCl was precipitated, and filtration was performed. The filtrate was vacuum-dried and crystallized to obtain yellow crystalline Compound 1 (2.4 g, yield 71%).

¹H NMR (500 MHz, Chloroform-*d*) δ 7.94 (d, *J* = 9.5Hz, 2H), 7.80 (d, *J =* 7.4Hz, 2H), 7.71 (d, *J =* 7.5Hz, 2H), 7.05∼7.34 (m, 18H), 6.28 (t, *J* = 7.5Hz, 2H), 6.19 (s, 2H), 5.28 (t, *J* = 7.4Hz, 2H), 1.42 (s, 6H), 1.38 (s, 6H)

### [Comparative Preparation Example 1] Synthesis of [1-(η5-Cyclopentadien-1-yl)-1-(η5-fluorenyl)-1,1-diphenyl methane]hafnium dichloro (Compound 2)

[1-(η5-Cyclopentadien-1-yl)-1-(η5-fluorenyl)-1,1-diphenyl methane]hafnium dichloro (Compound 2) was synthesized according to the preparation procedure of the document [A. Razavi, J. L. Atwood, J. Organometallic. Chen, 459 (1993), 117-123] .

¹H NMR (500 MHz, Chloroform-d) δ 8.19 (d, *J* = 8.5 Hz, 2H), 7.95 (d, *J* = 8.3 Hz, 2H), 7.88 (d, *J* = 8.5 Hz, 2H), 7.55 (t, *J* = 8.0 Hz, 2H), 7.44 (t, *J* = 8.2 Hz, 2H), 7.31 (m, 4H), 7.01 (t, *J* = 8.1 Hz, 2H), 6.47 (d, *J* = 7.1 Hz, 2H), 6.33 (s, 2H), 5.75 (s, 2H)

### [Comparative Preparation Example 2] Preparation of [1-(η5-Cyclopentadien-1-yl)-1-(η5-2,7-di-t-butylfluorenyl)-1,1-diphenyl methane]hafnium dichloro (Compound 3)

### Step 1: Preparation of 1-(2,4-Cyclopentadien-1-yl)-1-(2,7-di-t-butylfluorenyl)-1,1-diphenyl methane (Compound 3-a)

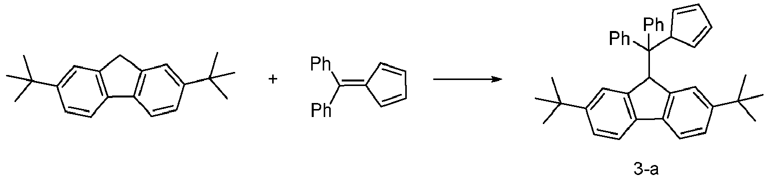

2,7-Di-t-butylfluorene (12 g, 43.1 mmol) was dissolved in 87 mL of THF, nBuLi (1.6 M in Hex, 27.1 mL, 43.1 mmol) was added, and stirring was performed at room temperature for 3 hours. 6,6-Diphenylfulvene (10 g, 43.1 mmol) was added, stirring was performed for 16 hours, and then the reaction was terminated by adding an aqueous NH₄Cl solution (40 mL). The product was extracted into an organic layer, the organic layer was dried over MgSO₄ and filtered under reduced pressure, and then the resulting yellow solid was washed with ethanol, thereby obtaining Compound 3-a (20g, yield 93%) as a white solid.

¹H NMR (500 MHz, Chloroform-d) δ 6.21∼7.35 (m, 20H), 5.45 (s, 1H), 3.01 (m, 1H), 1.14 (s, 18H)

### Step 2: Preparation of [1-(η5-Cyclopentadien-1-yl)-1-(η5-2,7-di-t-butylfluorenyl)-1,1-diphenyl methane]hafnium dichloro (Compound 3)

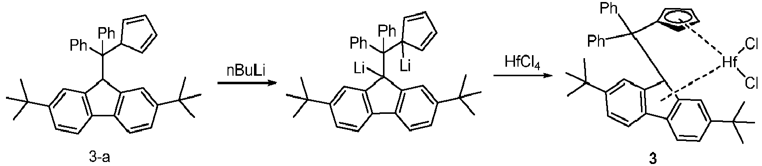

Compound 3-a (5 g, 9.82 mmol) was dissolved in 60 mL of ether, nBuLi (1.6 M in Hex, 13.5 mL, 21.6 mmol) was added, and stirring was performed for 16 hours. Ether was removed by vacuum drying, and then Hex was added and decanted under reduced pressure. Lithium (5.1 g, 9.79 mmol) and HfCl₄ (3.13 g, 9.79 mmol) that were weighed in a glove box were dissolved in 80 mL of ether, and then stirring was performed at room temperature for 16 hours. Ether was removed by vacuum drying, 80 mL of toluene was added, heating was performed at 50°C for 2 hours, produced LiCl was precipitated, and filtration was performed. The filtrate was vacuum-dried and crystallized to obtain yellow crystalline Compound 3 (4.4 g, yield 60%).

¹H NMR (500 MHz, Chloroform-*d*) δ 8.03 (d, *J* = 9.0 Hz, 2H), 7.97 (d, *J* = 2.5 Hz, 2H), 7.96 (d, *J* = 3.0 Hz, 2H), 7.57 (d, *J* = 9.0 Hz, 2H), 7.45 (m, 2H), 7.36 (m, 2H), 7.29 (m, 2H), 6.37 (s, 2H), 6.29 (t, *J* = 2.5 Hz, 2H), 5.63 (t, *J* = 3.0 Hz, 2H), 1.04 (s, 18H)

[Comparative Preparation Example 3] Preparation of [1-(η5-Cyclopentadien-1-yl)-1-(η5-2,7-di-phenylfluorenyl)-1,1-diphenyl methane]hafnium dichloro (Compound 4)

### Step 1: Preparation of 2,7-Di-phenylfluorene (Compound 4-a)

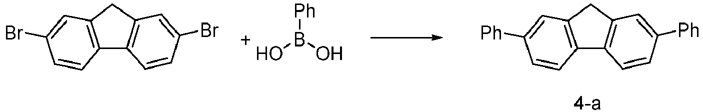

Pd(PPh₃)₄ (1.8 g, 1.54 mmol) and 2,7-dibromo-9H-fluorene (5 g, 15.4 mmol) were added to a Schlenk flask. 100 mL of toluene was added, phenylboronic acid (7.5 g, 61.7 mmol) was added, and 50 mL of toluene and an aqueous K₂CO₃ solution (30 mL, 2 M) were added. Reflux was performed at 100°C for 16 hours, water and ether were added, and then an organic layer was extracted three times. The organic layers were collected, dried over MgSO₄, and filtered under reduced pressure, and the resultant was purified by a silica column (Hex:Ethyl Acetate = 10:1), thereby obtaining Compound 4-a (3.1 g, yield 63%) as a white solid.

¹H NMR (500 MHz, Chloroform-d) δ 7.87 (d, *J* = 7.7 Hz, 2H), 7.79 (s, 2H), 7.67 (m, 6H), 7.46 (m, 4H), 7.36 (d, *J* = 7.7 Hz, 2H), 4.03 (s, 2H)

### Step 2: Preparation of 1-(2,4-Cyclopentadien-1-yl)-1-(2,7-di-phenylfluorenyl)-1,1-diphenyl methane (Compound 4-b)

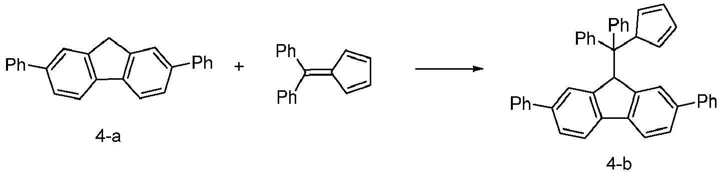

Compound 4-a (7 g, 21 mmol) was dissolved in 40 mL of THF, nBuLi (1.6 M in Hex, 14 mL, 21 mmol) was added, and stirring was performed at room temperature for 3 hours. 6, 6-Diphenylfulvene (5 g, 21 mmol) was added, stirring was performed for 16 hours, and then the reaction was terminated by adding an aqueous NH₄Cl solution (20 mL). The product was extracted into an organic layer, the organic layer was dried over MgSO₄ and filtered under reduced pressure, and then the resulting yellow solid was washed with ethanol, thereby obtaining Compound 4-b (20 g, yield 91%) as a white solid.

¹H NMR (500 MHz, Chloroform-d) δ 7.30∼7.73 (m, 30H), 5.61 (s, 1H), 4.09 (s, 1H)

### Step 3: Preparation of [1-(η5-cyclopentadien-1-yl)-1-(η5-2,7-di-phenylfluorenyl)-1,1-diphenyl methane]hafnium dichloro (Compound 4)

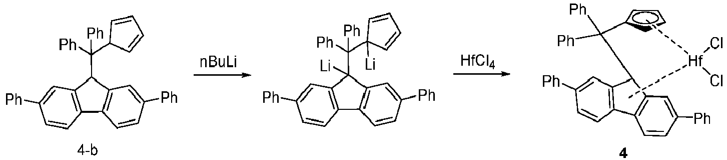

Compound 4-b (5 g, 9.11 mmol) was dissolved in 60 mL of ether, nBuLi (1.6 M in Hex, 13.5 mL, 21.6 mmol) was added, and stirring was performed for 16 hours. Ether was removed by vacuum drying, and then Hex was added and decanted under reduced pressure. Lithium (5.1 g, 9.11 mmol) and HfCl₄ (2.91 g, 9.11 mmol) that were weighed in a glove box were dissolved in 80 mL of ether. Stirring was performed at room temperature for 16 hours, ether was removed by vacuum drying, 80 mL of toluene was added, heating was performed at 50°C for 2 hours, produced LiCl was precipitated, and filtration was performed. The filtrate was vacuum-dried and crystallized to obtain yellow crystalline Compound 4 (10 g, yield 67%).

¹H NMR (500 MHz, Chloroform-d) δ 8.23 (d, *J* = 9.0 Hz, 2H), 7.96 (d, *J* = 2.5 Hz, 2H), 7.94 (d, *J* = 3.0 Hz, 2H), 7.78 (d, *J* = 9.0 Hz, 2H), 7.27∼7.48 (m, 16H), 6.60 (s, 2H), 6.28 (s, 2H), 5.74 (s, 2H)

[Example 1] Production of copolymer of ethylene and α-olefin

1 L of Hex and triisobutylaluminum (1 M, 2 mL) were added to a 4 L reactor at room temperature, and then 1-octene (100 mL) was added. Compound 1 (Preparation Example 1) (3.9 µmol) was dissolved in triisobutylaluminum (0.1 M in Hex, 2 mL) in a glove box, 5 mL of hexane was added, and then the solution was injected into an inlet of the reactor. Trityl tetrakis(pentafluorophenyl)borate (19.5 pmol) was dissolved in 5 mL of Hex in the glove box, and then the solution was injected into the inlet. The temperature of the reactor was raised to 140°C, and the solution was injected into the inlet with highpressure nitrogen. The injection of ethylene was performed at 300 psig for 15 minutes, and the initial temperature increased in proportion to the activity. After the polymerization reaction was completed, the temperature of the reactor was cooled to 30°C, and the ethylene pressure inside the reactor was slowly evacuated to remove ethylene. The produced polymer was washed with ethanol and acetone, and the washed polymer was filtered and vacuum-dried. The results of the produced polymers are shown in Table 1.

### [Example 2]

The same procedure as that of Example 1 was performed, except that anilinium tetrakis(pentafluorophenyl)borate was used instead of trityl tetrakis(pentafluorophenyl)borate. The results thereof are shown in Table 1.

### [Example 3]

The same procedure as that of Example 1 was performed, except that the reaction was performed at 150°C instead of 140°C. The results thereof are shown in Table 1.

### [Example 4]

The same procedure as that of Example 1 was performed, except that the reaction was performed at 150°C instead of 140°C, and anilinium tetrakis(pentafluorophenyl)borate was used instead of trityl tetrakis(pentafluorophenyl)borate. The results thereof are shown in Table 1.

### [Comparative Example 1]

The same procedure as that of Example 1 was performed, except that Compound 2 (Comparative Preparation Example 1) was used instead of Compound 1 (Preparation Example 1). The results thereof are shown in Table 1.

### [Comparative Example 2]

The same procedure as that of Example 1 was performed, except that Compound 2 (Comparative Preparation Example 1) was used instead of Compound 1 (Preparation Example 1), and anilinium tetrakis(pentafluorophenyl)borate was used instead of trityl tetrakis(pentafluorophenyl)borate. The results thereof are shown in Table 1.

### [Comparative Example 3]

The same procedure as that of Example 1 was performed, except that Compound 3 (Comparative Preparation Example 2) was used instead of Compound 1 (Preparation Example 1). The results thereof are shown in Table 1.

### [Comparative Example 4]

The same procedure as that of Example 1 was performed, except that Compound 3 (Comparative Preparation Example 2) was used instead of Compound 1 (Preparation Example 1), and anilinium tetrakis(pentafluorophenyl)borate was used instead of trityl tetrakis(pentafluorophenyl)borate. The results thereof are shown in Table 1.

### [Comparative Example 5]

The same procedure as that of Example 1 was performed, except that Compound 3 (Comparative Preparation Example 2) was used instead of Compound 1 (Preparation Example 1), and the reaction was performed at 150°C instead of 140°C. The results thereof are shown in Table 1.

### [Comparative Example 6]

The same procedure as that of Example 1 was performed, except that Compound 3 (Comparative Preparation Example 2) was used instead of Compound 1 (Preparation Example 1), anilinium tetrakis(pentafluorophenyl)borate was used instead of trityl tetrakis(pentafluorophenyl)borate, and the reaction was performed at 150°C instead of 140°C. The results thereof are shown in Table 1.

### [Comparative Example 7]

The same procedure as that of Example 1 was performed, except that Compound 4 (Comparative Preparation Example 3) was used instead of Compound 1 (Preparation Example 1). The results thereof are shown in Table 1.

### [Comparative Example 8]

The same procedure as that of Example 1 was performed, except that Compound 4 (Comparative Preparation Example 3) was used instead of Compound 1 (Preparation Example 1), and anilinium tetrakis(pentafluorophenyl)borate was used instead of trityl tetrakis(pentafluorophenyl)borate. The results thereof are shown in Table 1.

The analysis results of DSC and GPC of the copolymers of Example 1 and Comparative Examples 1, 3, and 7 obtained by using the same temperature and cocatalyst are shown in Table 2.

**[Table 1]**

| | Transition metal compound | Cocatalyst | Polymerization temperature (°C) | Activity (Kg/g cat) | MI (g/10 min) | | Density (g/mL) |
|---|---|---|---|---|---|---|---|
| | | | | | @2.16 Kg | @21.6 Kg | |
| Example 1 | Preparation Example 1 (Compound 1) | Trityl tetrakis(pentaflu orophenyl)borate | 140 | 11.1 | - | 1.7796 | 0.8649 |
| Example 2 | Preparation Example 1 (Compound 1) | Anilinium tetrakis(pentaflu orophenyl)borate | 140 | 12.7 | - | 2.708 | 0.8634 |
| Example 3 | Preparation Example 1 (Compound 1) | Trityl tetrakis (pentaflu orophenyl)borate | 150 | 7.3 | - | 7.8326 | 0.8632 |
| Example 4 | Preparation Example 1 (Compound 1) | Anilinium tetrakis(pentaflu orophenyl)borate | 150 | 8.6 | - | 9.7251 | 0.8626 |
| Comparative Example 1 | Comparative Preparation Example 1 (Compound 2) | Trityl tetrakis(pentaflu orophenyl)borate | 140 | 1.8 | 0.9142 | - | 0.8603 |
| Comparative Example 2 | Comparative Preparation Example 1 (Compound 2) | Anilinium tetrakis(pentaflu orophenyl)borate | 140 | 2.1 | 1.1282 | - | 0.8642 |
| Comparative Example 3 | Comparative Preparation Example 2 (Compound 3) | Trityl tetrakis(pentaflu orophenyl)borate | 140 | 10.1 | - | 7.7753 | 0.8612 |
| Comparative Example 4 | Comparative Preparation Example 2 (Compound 3) | Anilinium tetrakis(pentaflu orophenyl)borate | 140 | 12.1 | - | 8.2842 | 0.8630 |
| Comparative Example 5 | Comparative Preparation Example 2 (Compound 3) | Trityl tetrakis(pentaflu orophenyl)borate | 150 | 5.5 | - | 18.8583 | 0.8631 |
| Comparative Example 6 | Comparative Preparation Example 2 (Compound 3) | Anilinium tetrakis(pentaflu orophenyl)borate | 150 | 6.3 | - | 13.9496 | 0.8649 |
| Comparative Example 7 | Comparative Preparation Example 3 (Compound 4) | Trityl tetrakis(pentaflu orophenyl)borate | 140 | 0.4 | ND | | ND |
| Comparative Example 8 | Comparative Preparation Example 3 (Compound 4) | Anilinium tetrakis(pentaflu orophenyl)borate | 140 | 0.6 | ND | | ND |

**[Table 2]**

| | DSC | | | GPC | | |
|---|---|---|---|---|---|---|
| | Tc (°C) | Tm (°C) | ΔH (J/g) | Mw (x 10⁴) (g/mol) | Mn (x 10⁴) (g/mol) | MWD |
| Example 1 | 65.5/72.6 | 52.5/104.4 | -/2.8 | 26.6 | 12.3 | 2.16 |
| Comparative Example 1 | 89.4 | 54.9/108.8 | -/3.6 | 13.2 | 5.7 | 2.32 |
| Comparative Example 3 | 40.4/71.4 | 56.9/108.3 | 7.7/- | 15.9 | 7.61 | 2.09 |
| Comparative Example 7 | 39.3/96.8 | 56.5/115.9 | -/6.9 | 21.1 | 7.76 | 2.72 |

As shown in Table 1, in the production of the copolymer of ethylene and 1-octene, it could be appreciated that, in Examples 1 to 4 of the present invention, the catalytic activity was superior to that in each of Comparative Examples in which the compounds were changed under the same conditions.

In addition, as shown in Table 2, as a result of comparing the copolymers of ethylene and 1-octene of Example 1 and Comparative Examples 1, 3, and 7 produced under the same conditions, in Example 1 of the present invention, the weight average molecular weight and the number average molecular weight were 1.3 to 2.0 times and 1.6 to 2.2 times higher than those of other cases, respectively. Therefore, it can be appreciated that a polymer having a significantly high molecular weight may be produced when the transition metal compound of the present invention is used as a catalyst.

In addition, in Example 1 of the present invention, Tm was lower than those in Comparative Examples 1, 3, and 7. It can be appreciated that the copolymerizability is excellent when the transition metal compound of the present invention is used as a catalyst in that Tm is lower as the copolymerizability is more excellent as described above.

The transition metal compound of the present invention has a structure in which transition metals of Group 4 in the periodic table as central metals are linked by a cyclopentadienyl group in which electrons are rich and widely delocalized and a fluorenyl group substituted with an arylalkyl substituent at positions 2 and 7 that may stabilize an active site, such that excellent catalytic activity and high molecular weight may be exhibited in a high-temperature solution polymerization of ethylene and olefins.

Therefore, when the catalyst composition containing the transition metal compound of the present invention is used for producing a copolymer of ethylene and α-olefin, it is possible to produce a significantly improved high molecular weight copolymer with high yield, and it is expected that copolymers exhibiting excellent physical properties may be mass-produced industrially in a significantly economical manner.

As set forth above, the novel transition metal compound of the present invention may be easily prepared with high yield by a simple process under mild conditions, the transition metal compound, which is a single active site catalyst, and the catalyst composition containing the same have excellent thermal stability and thus may maintain excellent catalytic activity even at a high temperature, and a high molecular weight copolymer of ethylene and α-olefin may be produced using the transition metal compound. The method for producing a copolymer of ethylene and α-olefin is a significantly economical method because it is possible to obtain a copolymer having various physical properties with high yield by a simple process, and may be easily used for industrial mass production.

Hereinabove, although the present invention has been described by specific matters and limited Examples and Comparative Examples, they have been provided only for assisting in the entire understanding of the present invention. Therefore, the present invention is not limited to the Examples. Various modifications and changes may be made by those skilled in the art to which the present invention pertains from this description.

Therefore, the spirit of the present invention should not be limited to the described Examples, but the claims and all modifications equal or equivalent to the claims are intended to fall within the spirit of the present invention.

## Claims

1. A transition metal compound represented by the following Chemical Formula 1: 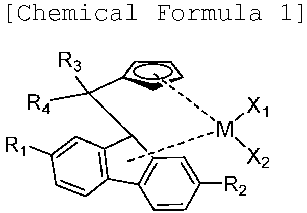 wherein
M is a Group 4 transition metal;
R₁ and R₂ are each independently (C6-C20)aryl(C1-C20)alkyl unsubstituted or substituted with (C1-C10)alkyl;
R₃ and R₄ are each independently (C6-C20)aryl unsubstituted or substituted with (C1-C10)alkyl;
X₁ and X₂ are each independently halogen, (C1-C20) alkyl, (C3-C20)cycloalkyl, (C6-C20)aryl, (C6-C20) aryl (C1-C20) alkyl, ((C1-C20)alkyl(C6-C20)aryl)(C1-C20)alkyl, (C1-C20)alkoxy, (C6-C20)aryloxy, (C1-C20)alkyl(C6-C20)aryloxy, (C1-C20)alkoxy(C6-C20) aryloxy, -OSiRₐR_{b}R_{c}, -SR_{d}, -NRₑR_{f}, -PR_{g}Rₕ, or (C1-C20)alkylidene;
Rₐ to R_{d} are each independently (C1-C20)alkyl, (C6-C20)aryl, (C6-C20)aryl(C1-C20)alkyl, (C1-C20)alkyl(C6-C20)aryl, or (C3-C20)cycloalkyl;
Rₑ to Rₕ are each independently (C1-C20)alkyl, (C6-C20)aryl, (C6-C20)aryl(C1-C20)alkyl, (C1-C20)alkyl(C6-C20)aryl, (C3-C20)cycloalkyl, tri(C1-C20)alkylsilyl, or tri(C6-C20)arylsilyl; and
when one of X₁ and X₂ is (C1-C20)alkylidene, the other one is absent.

2. The transition metal compound of claim 1, wherein in Chemical Formula 1,
M is Ti, Zr, or Hf;
R₁ and R₂ are each independently (C6-C20)aryl(C1-C20)alkyl; R₃ and R₄ are each independently (C6-C12)aryl unsubstituted or substituted with (C1-C5)alkyl; and
X₁ and X₂ are each independently halogen, (C1-C20) alkyl, (C6-C20)aryl, or (C6-C20)aryl(C1-C20)alkyl.

3. The transition metal compound of claim 1, wherein in Chemical Formula 1,
M is Hf;
R₁ and R₂ are each independently (C6-C12)aryl(C1-C10)alkyl;
R₃ and R₄ are each independently (C6-C12)aryl; and
X₁ and X₂ are each independently halogen, (C1-C10) alkyl, (C6-C12)aryl, or (C6-C12)aryl(C1-C10)alkyl.

4. The transition metal compound of claim 1, wherein in Chemical Formula 1,
M is Hf;
R₃ and R₄ are each independently phenyl;
X₁ and X₂ are each independently methyl, benzyl, or Cl; and
R₁ and R₂ are each independently represented by the following Chemical Formula 2: 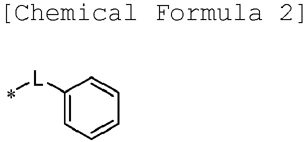 wherein
L is linear or branched (C1-C10)alkylene.

5. The transition metal compound of claim 1, wherein the transition metal compound is [1-(η5-cyclopentadien-1-yl)-1-(η5-2,7-di-(2-phenylpropan-2-yl)fluorenyl)-1,1-diphenyl methane]hafnium dichloro, [1-(η5-cyclopentadien-1-yl)-1-(η5-2,7-di-(2-phenylpropan-2-yl)fluorenyl)-1,1-diphenyl methane]hafnium dibenzyl, or [1-(η5-cyclopentadien-1-yl)-1-(η5-2,7-di-(2-phenylpropan-2-yl)fluorenyl)-1,1-diphenyl methane]hafnium dimethyl.

6. A transition metal catalyst composition for producing a copolymer of ethylene and α-olefin, comprising:
the transition metal compound of any one of claims 1 to 5; and
a cocatalyst selected from an aluminum compound, a boron compound, and a mixture thereof.

7. The transition metal catalyst composition of claim 6, wherein the aluminum compound used as the cocatalyst is one or two or more selected from aluminoxane and organic aluminum.

8. A method for producing a copolymer of ethylene and α-olefin, the method comprising:
a) mixing the transition metal catalyst composition of claim 6, ethylene, and an α-olefin comonomer; and
b) performing a copolymerization reaction at a temperature of 110 to 170°C.

9. The method of claim 8, wherein the α-olefin copolymerized with ethylene is one or two or more selected from propylene, 1-butene, 1-pentene, 4-methyl-1-pentene, 1-hexene, 1-heptene, 1-octene, 1-decene, 1-undecene, 1-dodecene, 1-tetradecene, 1-hexadecene, 1-octadecene, 1-eicosene, cyclopentene, cyclohexene, norbornene, phenylnorbornene, styrene, α-methylstyrene, p-methylstyrene, and 3-chloromethylstyrene.

10. The method of claim 8, wherein the step b) is performed at a temperature of 120 to 160°C and a pressure of 10 to 100 bar.

11. The method of claim 8, wherein the method for producing a copolymer of ethylene and α-olefin is performed in a C5-C12 aliphatic hydrocarbon solvent.

12. A copolymer of ethylene and α-olefin produced using the transition metal compound of any one of claims 1 to 5 as a catalyst.
